# EUROPEAN PATENT SPECIFICATION

(11) **EP 1 742 953 B1**
(45) Date of publication and mention of the grant of the patent: **09.04.2008**
(21) Application number: 05713729.1
(22) Date of filing: 17.02.2005
(51) Int. Cl.: C07D 519/02

(54) **PROCESS FOR ISOLATION OF ERGOT ALKALOIDS FROM ERGOT**
VERFAHREN ZUR ISOLIERUNG VON ERGOTALKALOIDEN AUS ERGOT
PROCEDE PERMETTANT D'ISOLER LES ALCALOIDES DE L'ERGOT

(30) Priority: 20.02.2004 US 546561 P
(43) Date of publication of application: 17.01.2007
(73) Proprietor: IVAX PHARMACEUTICALS S.R.O., 747 70 Opava 9 (CZ)
(72) Inventor: CVAK, Ladislav, 746 01 Opava (CZ); HOLAN, Jiri, 747 05 Opava 5 (CZ); RODER, Lubomir, 746 01 Opava (CZ)
(74) Representative: Gallagher, Kirk James
(86) International application number: PCT/US2005/005059
(87) International publication number: WO 2005/082910

(56) References cited:
- EP-A- 0 022 418
- GB-A- 809 912
- GB-A- 1 299 557

## Description

### FIELD OF THE INVENTION

The present invention relates to a process for the extraction and purification of ergot alkaloids and in particular to the extraction and purification of ergot peptide alkaloids from the fungi *Claviceps purpurea.*

### BACKGROUND OF THE INVENTION

Ergot peptide alkaloids, also called ergopeptines, are natural products used for the manufacture of drugs. They are known to have therapeutic value themselves (e.g., ergotamine) or in their hydrogenated form such as their dihydroderivatives, e.g., dihydroergotamine, dihydroergocristine, etc. Additionally, they are known starting compounds for the partial synthesis of some semisynthetic drugs, such as nicergoline, pergolide, etc.

Ergot alkaloids of the peptide type are produced by the fungi *Claviceps purpurea,* which can be cultivated under parasitic conditions (growing on fields using rye as the host plant) or saprophytic conditions (i.e., fermentation). Although the processes used for isolating ergopeptines from field ergot and from fermentation broth share common features (i.e., similar solvents and purification techniques), they differ substantially in the nature of the starting material.

Several processes for extraction of ergot have previously been described. Individual processes, mainly that used for large-scale isolation of ergot alkaloids, differ in the solvents that are used. For example, these older processes used aqueous ethanol or methanol (see DE 47 315 and DP</country><docnumber> 697 760</docnumber></plcit>), newer processes used chlorinated </country><docnumber> 697 760</docnumber></plcit>), newer processes used chlorinated 697 760), newer processes used chlorinated hydrocarbons (see DE 2 113 281, DE 2 637 764, and DD 10 059), diethylether (see CS 264 880 and CS 264 881), acetone (see DE 1 695 986), methyl isobutylketone (see BE 891 421) or ethylacetate (see DE 2 949 593 and EP 22 418). Some of these solvents, however, are not currently acceptable for large scale process due to safety and ecological concerns (diethylether and chlorinated hydrocarbons being examples). Some of these solvents are additionally not selective enough to produce ergot alkaloids with enough purity to be practical for manufacturing, e.g., aqueous methanol, aqueous ethanol, and acetone.

Ergot also contains up to 30% oil and other lipids. Previous processes that used solvents that extracted other components of ergot, mainly oil, and necessarily required an operation for the separation of alkaloids from the associated lipids. However, not all the known solvents are suitable for direct elimination of lipids by liquid-liquid extraction, and therefore the isolation procedures, including concentration of the primary extracts by evaporation and dissolving the residue in another solvent, added to the complexity of the process. Additionally, the evaporation of the primary extract containing oil and other ballast components is harmful to the extracted alkaloids.

Natural ergopeptines are derivatives of lysergic acid and readily isomerize to derivatives of isolysergic acid, the so-called ergopeptinines. This fact usually complicates the isolation of ergopeptines because the primary extracts obtained from ergot always contain mixtures of ergopeptines and ergopeptinines, which makes it difficult to obtain crystalline product, thus precluding crystallization techniques, which is an otherwise efficient means of purification. Therefore, processes for large-scale isolation of ergot alkaloids, using safe and environmentally friendly solvents with simple and efficient purification operations (e.g., crystallization) are still desirable.

### SUMMARY OF THE DISCLOSURE

The present invention provides a novel method of extracting ergot alkaloids from ergot.

The present method also provides a novel method of purifying the ergot extract providing ergot alkaloids in high yields and quality.

These and other advantages, which will become apparent during the following detailed description, have been obtained by the inventors' discovery that ergot can be extracted in high yield with a mixture of toluene and ethanol. Additional advantages include, but are not limited to, the ability to purify the ergot extract using relatively benign solvents and that the relatively simple process of the present invention can be scaled-up to industrial quantities.

### DETAILED DESCRIEPTION OF THE DISCLOSURE

The present invention is directed to the isolation of ergot alkaloids from ergot, i.e., *Claviceps purpurea,* by a simple, effective extraction process employing relatively safe and environmentally acceptable solvents. In one embodiment of the present invention, ergot is extracted with a toluene/ethanol mixture to form a primary extract. This primary extract can be further subjected to liquid-liquid extractions to further isolate and purify the ergot alkaloids obtained in the primary extract.

It is preferable that the toluene/ethanol extraction mixture comprises about 5, 6, 7, 8, 9, 10, 11, 12, 13, 14, 15, 16, 17, 18, 19, 20, 21, 22, 23, 24, 25, 26, 27, 28, 29, to 30% ethanol (v/v). When the concentration of ethanol is below 5%, then the extraction of ergot alkaloids from ergot is not as efficient. The upper amount of ethanol used is based on the intended selectivity of the extraction. For example, the more ethanol present the more polar ballast components that are extracted, and the more difficult it is to further process the primary extract. It is more preferable for 10-20% ethanol (v/v) to be present in the extraction mixture.

The temperature of the extraction process is advantageously not limited, because the usually unwanted isomerization of ergopeptines, which can occur at high temperature, does not impede the crystallization of the desired product. Nevertheless, temperature higher than about 50°C is not as economical. In a preferred embodiment, ergot is extracted with a toluene/ethanol mixture within a temperature range of between about 20, 25, 30, 35, 40, 45, to 50°C, with ambient temperature being preferred.

In another embodiment, the primary extract is subjected to liquid-liquid extraction using an aqueous solution of an acid. This liquid-liquid extraction of the primary extract advantageously permits separation of the generally more polar and hydrophilic alkaloids from the less polar and hydrophobic oils and lipids. Thus, liquid-liquid extraction of the primary extract with an aqueous solution containing an acid results in transferring the ergot alkaloids into the aqueous solution while leaving oil in the primary extract. The now formed aqueous extract, which contains alkaloids, can then be easily separated from the primary extract and further aids in the isolation and purification of the alkaloids. Any acid can be used with the aqueous solution. Hydrochloric acid is preferable due to the high solubility of ergopeptines hydrochlorides in aqueous hydrochloric acid solutions.

In another embodiment, an alcohol can be added to the aqueous solution to prevent or reduce the formation of an emulsion therein. It was discovered that under certain conditions, the aqueous solution can form an emulsion, which reduces the ability to isolate alkaloids. Hence, an emulsion inhibiting amount of alcohol, or similarly functioning solvent, can be added to the aqueous solution to enhance results. For example, ethanol can be added to an aqueous solution containing hydrochloric acid used for extraction of alkaloids from the primary extract. The concentration of acid in the aqueous solution is not critical, but the solution preferably contains at least one equivalent of acid to extract the alkaloids from the primary extract quantitatively. For example, the aqueous solution of acid preferably comprises from about 30% to 60% (v/v) of water, about 70 to 40% (v/v) of ethanol and about 0.05 to 1.0% (w/w) of acid. The aqueous solution of acid more preferably comprises from about 40-50% (v/v) of water, about 60-50% (v/v) of ethanol and about 0.1-0.3% (w/w) of acid The aqueous solution of acid even more preferably comprises from about 50% (v/v) of water, about 50% (v/v) of ethanol and about 0.2% (w/w) of acid or about 40% (v/v) of water, about 60% (v/v) of ethanol and about 0.2% (w/w) of acid.

In another embodiment, the obtained aqueous extract is made alkaline (e.g., pH>7.0), thereby facilitating the alkaloid's transfer into an organic solvent during another liquid-liquid extraction. This can be done with any aqueous alkaline solution as, for example, with an aqueous solution of sodium hydroxide, more preferably 5% aqueous sodium hydroxide (w/w).

In another embodiment, the aqueous extract, after its alkalinity has been raised above 7.0, is subjected liquid-liquid extraction with toluene. The toluene extract resulting from this liquid-liquid extraction step contains practically only ergot alkaloids, and therefore it is denominated as the purified toluene extract.

In another embodiment, the purified toluene extract is partially evaporated. This partial evaporation is intended to cause formation of a crystalline product. It was found that some alkaloids, namely ergotamine and ergocristine, can be obtained as crystalline products by merely evaporating the toluene extract. The fact that the organic solvent may contain a mixture of ergopeptine and ergopeptinine does not adversely influenced the crystallization of these products, because a crystalline mixture of corresponding ergopeptine and ergopeptinine, e.g., the mixture of ergotamine and ergotaminine or the mixture of ergocristine and ergocristinine, is obtained. Without being bound to any theory, it appears that the extraction and purification techniques of the present invention result in the isolation of ergot alkaloids in substantially pure form and without crystallization inhibiting impurities. Thus, even when there is a mixture of alkaloids extracted, a crystalline product can be obtained after partial evaporation of the solvent. Moreover, potential transformations of the ergot alkaloids such as isomerization of ergot alkaloids, which can occur at high temperature, does not appear to influence the isolation and crystallization of the products obtained after evaporation of the solvent. Hence, extraction at high temperatures does not adversely affect the present process. The crystallization of alkaloids from toluene has surprisingly provides high purity products as is demonstrated in Examples 1 and 2 by comparison of the alkaloid composition of the first and the second crops.

Under certain circumstances it was found that the toluene extract can contain a mixture of alkaloids *e*.*g*., a mixture of ergotoxine alkaloids, which do limit their ability to crystallize. The alkaloid composition of the toluene extract corresponds to the spectrum of alkaloids produced by the used strain of ergot. When such a mixture of ergotoxine alkaloids is present in the toluene extract, little or no crystalline product can be obtained directly from toluene. In another embodiment of the present invention, this limitation is overcome by the addition of one or more aliphatic hydrocarbon, *e*.*g*., a C₅-C₈ hydrocarbon such as hexane or heptane, to the solution. Hexane is a more preferred hydrocarbon. Such crystallization technique has no effect on the alkaloid composition, but it can still produce a crystalline product free of ballast components and suitable for further use as is demonstrated in Example 3.

Other features of the invention will become apparent in the course of the following descriptions of exemplary embodiments that are given for illustration of the invention and are not intended to be limiting thereof.

### EXAMPLES

### Example 1

About 20,000 kg of ergot (ergocristine strain GAL 130) were extracted under counter current conditions by a mixture of toluene and ethanol 87:13 (v/v) on a continual extractor of a carrousel type. Approximately 64 m³ of primary extract was obtained. The primary extract was then extracted on a continual extractor Westfalia by a mixture of ethanol and water 1:1 (v/v), containing 0.2% (w/w) hydrogen chloride. Approximately 28 m³ of aqueous extract resulted. The aqueous extract was made alkaline to about pH 7.3 by a 5% (w/w) aqueous sodium hydroxide solution and extracted with toluene on a continual extractor Westfalia. About 16 m³ of toluene extract was received. The toluene extract was evaporated to about 1000 kg and the resulting crystalline product was filtered off, washed with toluene and dried for 3 hours in a vacuum dryer at 60°C and 50 mbar, to obtain 152 kg of a First crop of Crude ergocristine. The mother liquors were evaporated to about 400 kg and 1500 L of technical hexane was added. The precipitated crystalline product was filtered off, washed with technical hexane and dried, to obtain 89 kg of a Second crop of Crude ergocristine.

Analytical results:

| | First crop | Second crop |
|---|---|---|
| Assay by titration | 93.1 % | 92.6% |
| Ergocristine | 30.9% | 20.0% |
| Ergocristinine | 59.3% | 36.6% |
| α-ergokryptine | 2.4% | 10.5% |
| α-ergokryptinine | 3.9% | 17.2% |
| Sum of other alkaloids | 2.6% | 15.7% |

### Example 2

About 20,000 kg of ergot (ergotamine strain GAL 404) was extracted under counter current conditions with a mixture of toluene and ethanol 84:16 (v/v) on a continual extractor of a carrousel type, and 78 m³ of primary extract was obtained. The primary extract was extracted on a continual extractor Westfalia by a mixture of ethanol and water 6:4 (v/v), containing 0.2% (w/w) hydrogen chloride and 18 m³ of aqueous extract resulted. The aqueous extract was made alkaline by increasing its pH to 7.3 by the addition of 5% (w/w) aqueous sodium hydroxide. It was then extracted with toluene on continual extractor Westfalia. 30 m³ of toluene extract was received. The toluene extract was evaporated to about 1000 kg and the resulting crystalline product was filtered off, washed with toluene, and dried for 3 hours in vacuum dryer at 60°C and 50 mbar, resulting in about 181 kg of a First crop of Crude ergotamine. The mother liquors were evaporated to about 100 kg and the crystalline product was filtered off, washed with toluene, and dried, thereby obtaining 19 kg of Second crop of Crude ergotamine.

Analytical results:

| | First crop | Second crop |
|---|---|---|
| Assay by titration | 95.0% | 90.6% |
| Ergotamine | 24.7% | 31.5% |
| Ergotaminine | 71.7% | 51.5% |
| Sum of other alkaloids | 3.6% | 17.0% |

### Example 3

About 20,000 kg of ergot (ergotoxine strain GAL 310) were extracted under counter current conditions with a mixture of toluene and ethanol 87:13 (v/v) on a continual extractor of a carrousel type. Approximately 69 m³ of primary extract was obtained. The primary extract was extracted on a continual extractor Westfalia by a mixture of ethanol and water 1:1 (v/v), containing 0.2% (w/w) hydrogen chloride resulting in 27 m³ of aqueous extract. The aqueous extract was made alkaline by increasing the pH of the aqueous extract to about 7.3 with 5% (w/w) aqueous sodium hydroxide. It was then extracted with toluene on a continual extractor Westfalia with about 17 m³ of toluene extract being received. The toluene extract was evaporated to about 800 kg and 1800 L of technical hexane was added. The precipitated crystalline product was filtered off, washed with technical hexane, and dried for 3 hours in vacuum dryer at 60°C and 50 mbar obtaining 151 kg of Crude ergotoxine.

Analytical results:

| | |
|---|---|
| Assay by titration | 94.4% |
| Ergocornine | 18.1% |
| Ergocorninine | 29.2% |
| α-ergokryptine | 11.7% |
| α-ergokryptinine | 18.5% |
| β-ergokryptine | 6.3% |
| β-ergokryptinine | 11.6% |
| Sum of other alkaloids | 4.6% |

## Claims

1. A process of isolating an ergot alkaloid from ergot, the process comprising: extracting ergot with a mixture, comprising: toluene and ethanol to form a primary extract.

2. The process of claim 1, wherein the mixture, comprises: toluene and 5-30% (v/v) of ethanol, preferably the mixture, comprises: toluene and 10-20% (v/v) of ethanol.

3. The process of claim 1 or claim 2, wherein the extracting is performed at a temperature of 20-50°C, preferably the extracting is performed at ambient temperature.

4. The process of claim 2, wherein the extracting is performed in a counter current way on a battery of percolators or on a continuous extractor.

5. The process of claim 2 or claim 3, further comprising: extracting the primary extract with an aqueous solution of an acid, such as hydrochloric acid, to transfer the ergot alkaloid from the primary extract to an aqueous extract.

6. The process of claim 5, wherein the aqueous solution of hydrochloric acid, comprises: 30-60% (v/v) water, 70-40% (v/v) ethanol, and 0.05-1. 0% (w/w) HCl, preferably the aqueous solution of hydrochloric acid, comprises: 40-50% (v/v) water, 60-50% (v/v) ethanol, and 0.1-0.3% (w/w) HCl.

7. The process of any of claims 2, 3, 5 or 6, further comprising: increasing the pH of the aqueous extract to above 7. 0.

8. The process of Claim 7, wherein the increasing is performed by the addition of an aqueous sodium hydroxide solution (w/w), preferably by the addition of a 5% aqueous sodium hydroxide solution (w/w).

9. The process of claim 7 or claim 8, further comprising: extracting the aqueous extract having a pH above 7.0 with toluene to transfer the ergot alkaloid from the aqueous solution and obtain a purified toluene extract.

10. The process of claim 9, further comprising: partially evaporating the solvent from the purified toluene extract to form crystalline ergot alkaloid.

11. The process of claim 10, further comprising: separating the crystalline ergot alkaloid from the remaining toluene.

12. The process of claim 10, further comprising: adding one or more C₅-C₈ aliphatic hydrocarbons to the concentrate after partial evaporation of toluene to aid in crystallizing the ergot alkaloid.

13. The process of claim 12, wherein the one or more aliphatic C₅-C₈ hydrocarbons are selected from hexane and heptane, preferably the aliphatic hydrocarbon is hexane.

14. The process of claims 12 or 13, further comprising: separating the crystalline ergot alkaloid from the toluene/aliphatic hydrocarbon mixture.

15. The process of claim 14, comprising isolating the crystalline ergot alkaloid in greater than 90% purity.

16. A process of isolating an ergot alkaloid from ergot, the process comprising:
a. extracting ergot with a mixture, comprising: toluene and ethanol to form a primary extract, wherein the mixture, comprises: toluene and 5-30% (v/v) of ethanol;
b. extracting the primary extract with an aqueous solution of an acid to transfer the ergot alkaloid from the primary extract to an aqueous extract;
c. increasing the pH of the aqueous extract to above 7.0;
d. extracting the aqueous extract having a pH above 7.0 with toluene to transfer the ergot alkaloid from the aqueous solution and obtain a purified toluene extract;
e. partially evaporating the solvent from the purified toluene extract to form crystalline ergot alkaloid;
f. optionally adding one or more C₅-C₈ aliphatic hydrocarbons to the concentrate after partial evaporation of toluene to aid in crystallizing the ergot alkaloid; and,
g. separating the crystalline ergot alkaloid from the toluene or toluene /aliphatic hydrocarbon mixture.

## Patentansprüche

1. Verfahren zum Isolieren eines Mutterkornalkaloids aus Mutterkorn, wobei das Verfahren umfaßt, daß man Mutterkorn mit einem Gemisch, umfassend Toluen und Ethanol, unter Bildung eines primären Extrakts extrahiert.

2. Verfahren nach Anspruch 1, wobei das Gemisch Toluen und 5-30% (v/v) Ethanol umfaßt und das Gemisch vorzugsweise Toluen und 10-20% (v/v) Ethanol umfaßt.

3. Verfahren nach Anspruch 1 oder Anspruch 2, wobei das Extrahieren bei einer Temperatur von 20-50°C erfolgt und das Extrahieren vorzugsweise bei Umgebungstemperatur erfolgt.

4. Verfahren nach Anspruch 2, wobei das Extrahieren nach dem Gegenstromprinzip auf einer Gruppe von Perkolatoren oder auf einem kontinuierlich arbeitenden Extraktor durchgeführt wird.

5. Verfahren nach Anspruch 2 oder Anspruch 3, welches weiterhin das Extrahieren des primären Extrakts mit einer wäßrigen Lösung einer Säure, wie Salzsäure, umfaßt, um das Mutterkornalkaloid von dem primären Extrakt auf einen wäßrigen Extrakt zu übertragen.

6. Verfahren nach Anspruch 5, wobei die wäßrige Lösung von Salzsäure 30-60% (v/v) Wasser, 70-40% (v/v) Ethanol und 0,05-1,0% (w/w) HCl umfaßt und die wäßrige Lösung von Salzsäure vorzugsweise 40-50% (v/v) Wasser, 60-50% (v/v) Ethanol und 0,1-0,3% (w/w) HCI umfaßt.

7. Verfahren nach einem der Ansprüche 2, 3, 5 oder 6, welches weiterhin das Erhöhen des pH-Werts des wäßrigen Extrakts auf über 7,0 umfaßt.

8. Verfahren nach Anspruch 7, wobei das Erhöhen durch Zugabe einer wäßrigen Natriumhydroxidlösung (w/w) erfolgt und vorzugsweise durch Zugabe einer 5%-igen wäßrigen Natriumhydroxidlösung (w/w) erfolgt.

9. Verfahren nach Anspruch 7 oder Anspruch 8, welches weiterhin das Extrahieren des wäßrigen Extrakts mit einem pH-Wert von größer als 7,0 mit Toluen umfaßt, um das Mutterkornalkaloid von der wäßrigen Lösung zu übertragen und einen gereinigten Toluenextrakt zu erhalten.

10. Verfahren nach Anspruch 9, welches weiterhin das teilweise Verdampfen des Lösungsmittels aus dem gereinigten Toluenextrakts unter Bildung von kristallinem Mutterkornalkaloid umfaßt.

11. Verfahren nach Anspruch 10, welches weiterhin das Abtrennen des kristallinen Mutterkornalkaloids von dem verbleibenden Toluen umfaßt.

12. Verfahren nach Anspruch 10, welches weiterhin umfaßt, daß man nach dem teilweisen Verdampfen von Toluen einen oder mehrere aliphatische C₅-C₈-Kohlenwasserstoffe zu dem Konzentrat zugibt, um die Kristallisation des Mutterkornalkaloids zu unterstützen.

13. Verfahren nach Anspruch 12, wobei der eine oder die mehreren aliphatischen C₅-C₈-Kohlenwasserstoffe unter Hexan und Heptan ausgewählt sind und der aliphatische Kohlenwasserstoff vorzugsweise Hexan ist.

14. Verfahren nach Anspruch 12 oder 13, welches weiterhin das Abtrennen des kristallinen Mutterkornalkaloids von dem Gemisch aus Toluen/aliphatischem Kohlenwasserstoff umfaßt.

15. Verfahren nach Anspruch 14, welches das Isolieren des kristallinen Mutterkornalkaloids in einer Reinheit von mehr als 90% umfaßt.

16. Verfahren zum Isolieren eines Mutterkornalkaloids aus Mutterkorn, wobei das Verfahren folgendes umfaßt:
a. Extrahieren von Mutterkorn mit einem Gemisch, welches Toluen und Ethanol umfaßt, unter Bildung eines primären Extrakts, wobei das Gemisch Toluen und 5-30% (v/v) Ethanol umfaßt,
b. Extrahieren des primären Extrakts mit einer wäßrigen Lösung einer Säure, um das Mutterkomalkaloid von dem ersten Extrakt auf einen wäßrigen Extrakt zu übertragen,
c. Erhöhen des pH-Werts des wäßrigen Extrakts auf über 7,0,
d. Extrahieren des wäßrigen Extrakts mit einem pH-Wert von größer als 7,0 mit Toluen, um das Mutterkornalkaloid von der wäßrigen Lösung zu übertragen und einen gereinigten Toluenextrakt zu erhalten,
e. teilweises Verdampfen des Lösungsmittels aus dem gereinigten Toluenextrakt unter Bildung von kristallinem Mutterkornalkaloid,
f. optional Zugeben eines oder mehrerer aliphatischer C₅-C₈-Kohlenwasserstoffe zu dem Konzentrat nach dem teilweisen Verdampfen von Toluen, um die Kristallisation des Mutterkornalkaloids zu unterstützen, und
g. Abtrennen des kristallinen Mutterkornalkaloids von dem Toluen oder dem Gemisch aus Toluen/aliphatischem Kohlenwasserstoff.

## Revendications

1. Procédé d'isolement d'un alcaloïde de l'ergot à partir d'ergot, le procédé comprenant l'étape consistant à : extraire l'ergot avec un mélange, comprenant : le toluène et l'éthanol pour former un extrait primaire.

2. Procédé selon la revendication 1, dans lequel le mélange comprend : le toluène et 5 à 30% (v/v) d'éthanol, de préférence le mélange comprend : le toluène et 10 à 20% (v/v) d'éthanol.

3. Procédé selon la revendication 1 ou 2, dans lequel l'extraction est réalisée à une température allant de 20°C à 50°C, de préférence l'extraction est réalisée à température ambiante.

4. Procédé selon la revendication 2, dans lequel l'extraction est réalisée à contre-courant sur une batterie de percolateurs ou sur un extracteur continu.

5. Procédé selon la revendication 2 ou 3, comprenant en outre l'étape consistant à : extraire l'extrait primaire avec une solution aqueuse d'un acide, telle que l'acide chlorhydrique, pour transférer l'alcaloïde de l'ergot, de l'extrait primaire vers un extrait aqueux.

6. Procédé selon la revendication 5, dans lequel la solution aqueuse d'acide chlorhydrique, comprend : 30 à 60% (v/v) d'eau, 70 à 40% (v/v) d'éthanol et 0,05 à 1,0% (p/p) de HCI, de préférence la solution aqueuse de l'acide chlorhydrique, comprend : 40 à 50% (v/v) d'eau, 60 à 50% (v/v) d'éthanol et 0,1 à 0,3% (p/p) de HCI.

7. Procédé selon l'une quelconque des revendications 2, 3, 5 ou 6, comprenant en outre l'étape consistant à : augmenter le pH de l'extrait aqueux à plus de 7,0.

8. Procédé selon la revendication 7, dans lequel l'augmentation est réalisée par l'addition d'une solution aqueuse d'hydroxyde de sodium (p/p), de préférence par l'addition d'une solution aqueuse à 5% d'hydroxyde de sodium (p/p).

9. Procédé selon la revendication 7 ou 8, comprenant en outre l'étape consistant à : extraire l'extrait aqueux ayant un pH supérieur à 7,0 avec le toluène pour transférer l'alcaloïde de l'ergot de la solution aqueuse et obtenir un extrait de toluène purifié.

10. Procédé selon la revendication 9, comprenant en outre l'étape consistant à : évaporer partiellement le solvant de l'extrait de toluène purifié pour former un alcaloïde cristallin de l'ergot.

11. Procédé selon la revendication 10, comprenant en outre l'étape consistant à : séparer l'alcaloïde cristallin de l'ergot du toluène restant.

12. Procédé selon la revendication 10, comprenant en outre l'étape consistant à : ajouter un ou plusieurs hydrocarbures aliphatiques en C₅ à C₈ au concentré après évaporation partielle du toluène pour faciliter la cristallisation de l'alcaloïde de l'ergot.

13. Procédé selon la revendication 12, dans lequel les hydrocarbures plus ou moins aliphatiques en C₅ à C₈ sont choisis parmi l'hexane et l'heptane, de préférence l'hydrocarbure aliphatique est l'hexane.

14. Procédé selon la revendication 12 ou 13, comprenant en outre l'étape consistant à : séparer l'alcaloïde cristallin de l'ergot du mélange toluène/hydrocarbure aliphatique.

15. Procédé selon la revendication 14, comprenant l'étape consistant à isoler l'alcaloïde cristallin de l'ergot avec une pureté supérieure à 90%.

16. Procédé d'isolement d'un alcaloïde de l'ergot à partir d'ergot, le procédé comprenant l'étape consistant à :
a. extraire l'ergot avec un mélange, comprenant : le toluène et l'éthanol pour former un extrait primaire, dans lequel le mélange comprend : le toluène et 5 à 30% (v/v) d'éthanol ;
b. extraire l'extrait primaire avec une solution aqueuse d'un acide pour transférer, de l'extrait primaire vers un extrait aqueux, l'alcaloïde de l'ergot ;
c. augmenter le pH de l'extrait aqueux à plus de 7,0 ;
d. extraire l'extrait aqueux ayant un pH supérieur à 7,0 avec le toluène pour transférer l'alcaloïde de l'ergot à partir de la solution aqueuse et obtenir un extrait purifié de toluène ;
e. évaporer partiellement le solvant de l'extrait purifié de toluène pour former un alcaloïde cristallin de l'ergot ;
f. ajouter éventuellement un ou plusieurs hydrocarbures aliphatiques en C₅ à C₈ au concentré après évaporation partielle du toluène pour faciliter la cristallisation de l'alcaloïde de l'ergot ; et
g. séparer l'alcaloïde cristallin de l'ergot du toluène ou du mélange toluène/hydrocarbure aliphatique.
